# EUROPEAN PATENT APPLICATION

(11) **EP 1 749 481 A1**
(43) Date of publication of application: **07.02.2007**
(21) Application number: 05425574.0
(22) Date of filing: 02.08.2005
(51) Int. Cl.: A61B 17/04

(54) **Suture device**

(71) Applicant: Gandini, Marco, 10040 Almese (Torino) (IT)
(72) Inventor: Gandini, Marco, 10040 Almese (Torino) (IT)
(74) Representative: Freyria Fava, Cristina

(57) **Abstract**

The present invention concerns suturing devices, including a T-shaped terminal anchor (85) having a central portion to which one end of a suture thread (S) is fixed, at least one intermediate anchor (8) having a shape elongated along a longitudinal axis (A) where this intermediate anchor (8) is provided with at least one through hole (81) transversal with respect to the longitudinal axis (A) and through which the suture thread (S) extends and can run and a locking device (50, 51, 52) to which the free end of the suture thread (S) is anchored.

## Description

The present invention concerns devices for suturing tissues, their relative locking devices and instruments for applying sutures. The invention can be used for example to close surgical incisions or repair wounds in a human or animal body.

Suturing is a surgical technique whereby two flaps of tissue are approximated and maintained in place for a sufficient period of time to enable the two flaps to cicatrise. This may be achieved through traditional methods using needles and threads of various shapes and materials, or with various suturing devices. For this purpose, different suturing devices have been conceived; these are known as anchors and essentially consist in a small bar having a longitudinal configuration to which is attached a suture thread that can be used to join the tissue flaps. To further facilitate the surgeon's work, suture locking devices of various shapes have been designed, that enable the stitch to be fastened without needing to be knotted. Examples of suture anchors may be found in international patent application WO-A-97/23157 and in US patent application US-A-2004/0092969.

The present invention has the purpose of enabling the surgeon to insert sutures in a continuous fashion, so as to close openings in tissues simply and rapidly without needing to make knots in the suture thread to fasten individual stitches.

This purpose is achieved thanks to new suturing devices enabling the continuous suturing of tissues and/or repair of surgical incisions thanks to the relative locking devices with which the stitches can be fastened without using knots.

In accordance with the present invention, this purpose is achieved thanks to the solution that will be described specifically in the attached claims. The claims are an integral part of the technical instruction provided here in regard to the invention.

The invention will now be described in detail, as a simple and non-restrictive example, with reference to the attached figures, in which:
-- figure 1 illustrates a suture anchor as per the present invention, in section;
-- figure 2 illustrates a first embodiment of a locking device as per the present invention, in section;
-- figures 3, 4, and 5 illustrates a second embodiment of a suture locking device as per the present invention, respectively in plan, perspective and section;
-- figure 6 illustrates a variant of the second embodiment of suture locking device as per the present invention, in section;
-- figures 7 and 8 illustrates a third embodiment of a suture locking device as per the present invention, respectively in perspective and in plan;
-- figures 9 and 10 represents, in diagram form, two methods to apply continuous sutures through T-shaped anchors with relative enlarged details;
-- figures 11 to 14 illustrate, respectively, two views, a section and an enlarged detail of a portion of the instrument to apply sutures as per a first embodiment of the present invention;
-- figure 15 illustrates the plan of an enlarged detail of the instrument to apply sutures as per the present invention;
-- figure 16 represents a wound in diagram form. **T-shaped suture anchor**

The T-shaped suture device, in short known as "anchor", comprises a cylindrical structure, approximately comparable to a spindle, at whose centre suture thread can be a attached. This type of configuration is generally known and marketed as a "Brown-Mueller T-fastener".

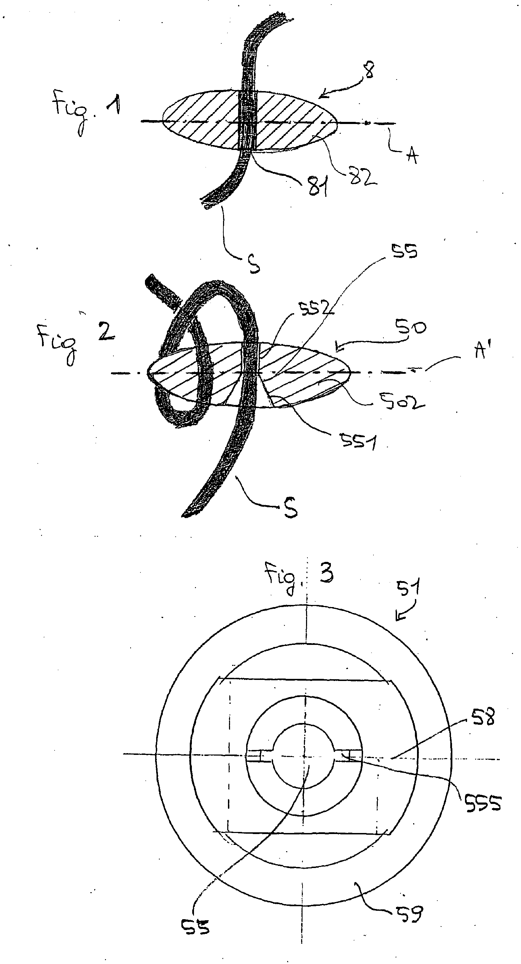

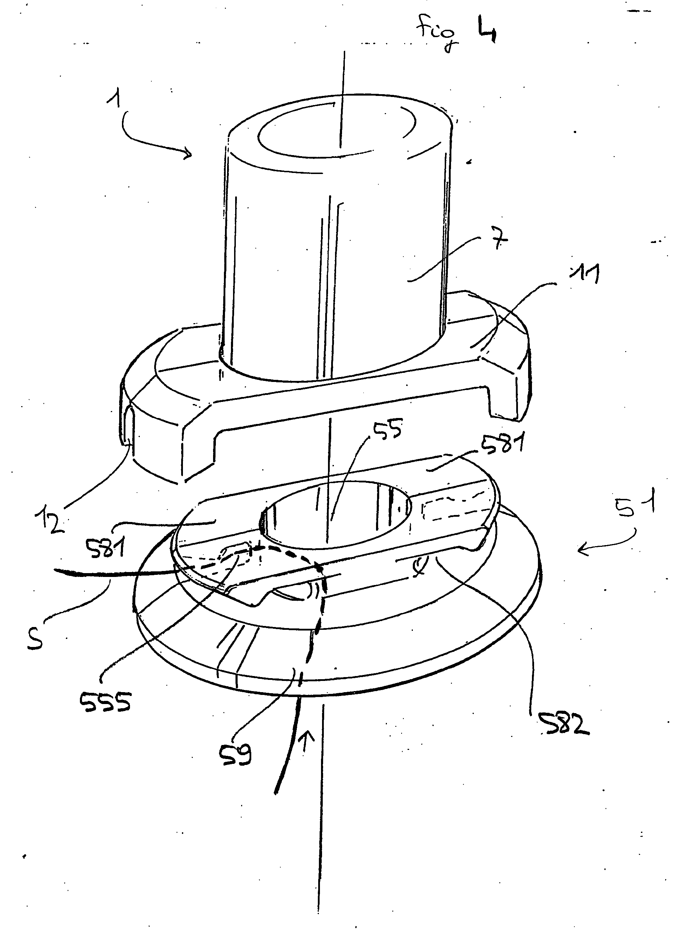

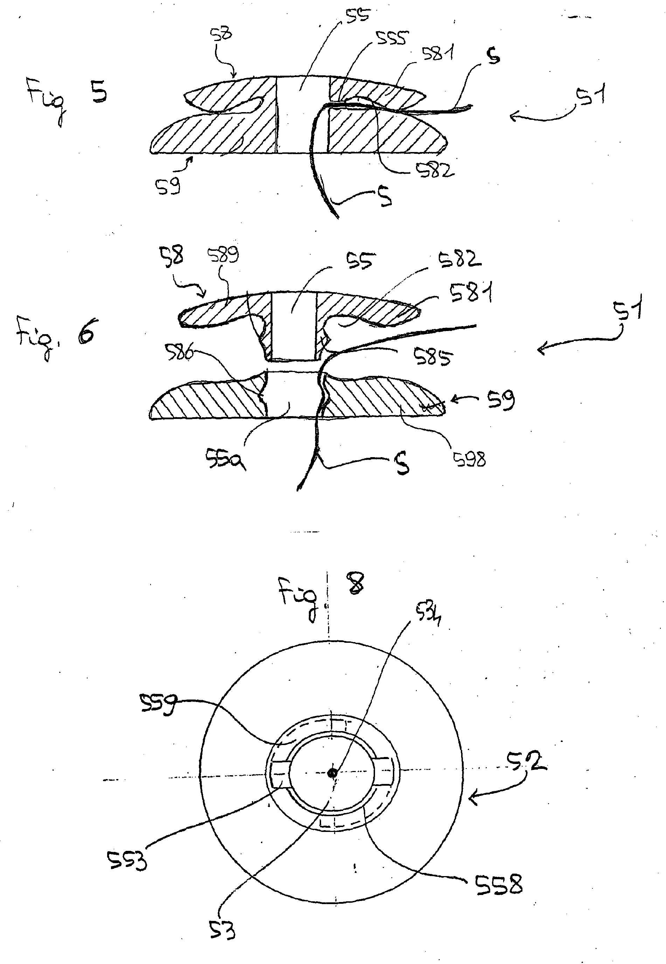

The suture anchor 8 subject of the present invention, presents at its centre a through hole 81 transversal to the longitudinal axis of the anchor 8 (figure 1).

The suture thread S passes freely through the hole 81. The advantage of this conformation is that the thread can run freely, allowing continuous sutures to be applied.

By means of the instrument 1 to apply sutures, which will be described below, the anchors 8 are inserted in the margin of the break in the tissue to be sutured.

Generally, suturing techniques provide for the use of a suture anchor of the known type as first anchor, that is without a through hole, but attached to the suture thread in a fixed fashion, while the subsequent anchors 8 each present a through hole 81.

Making reference to figure 16 which illustrates in diagram form a surgical wound with two flaps L1 and L2, reference numbers 71 and 74 indicate the outer surfaces, and reference numbers 75 and 76 indicate the inner surfaces, respectively, of the two flaps of the wound, L1 and L2. The margins of the wound are indicated with reference numbers 72 and 73, respectively, of flaps L1 and L2.

A wound may be sutured following two different modalities: the first modality entails approximating flaps L1 and L2 of the wound and bringing into contact the inner surfaces 75 and 76 so as to leave the margins 72 and 73 of the wound exposed outwards - the extroflected suture -- is illustrated in figure 9. The other modality for suturing a wound consists in approximating the margins 72 and 73 as illustrated in figure 10 - this is known as an introflected suture.

To apply continuous sutures, an anchor of known type 85 is used initially, fastened to the suture thread S.

With reference to figure 9, the anchor 85 is inserted using a specific suturing instrument, 1, through flap L2 and flap L1, so as to release it on the outer surface 71 of flap L1, and the suture thread S is made to pass through the two flaps so as to approximate them, coming out through the surface 74 of flap L2. Subsequently, an anchor with through hole 8 is inserted through the two flaps and again positioned on the outer surface 71 of flap L1. The suture thread S is again made to pass through the two flaps, L1 and L2 and pulled through the anchor 8 so as to approximate the two flaps, taking a new stitch a little below the first one and thus leaving the margins 72 and 73, of the wound exposed outwards. The necessary number of anchors 8 to completely close the wound are inserted sequentially. The use of anchors with a through hole makes it easier to pull the suture thread S through the flaps of the wound, and facilitates the suturing process. This type of suture is known as a continuous everting suture.

A different type of suture, known as an inverting suture, is illustrated in figure 10, where the anchors with through hole 8 are alternately inserted in flap L1 and in flap L2, producing a sort of zigzag by means of the suture thread S which approximates the two flaps. In this case, too, the first anchor used is an anchor of known type 85 fastened to the suture thread, and only the subsequent anchors have through holes, enabling the thread to be pulled through them and consequently to approximate the two flaps L1 and L2. More in particular the first anchor 85 is inserted in flap L1 and released on the inner surface 75 of flap L1; the suture thread S comes out through the outer surface 71; and a second anchor 8 is inserted in flap L2 and released on the surface 76. The third anchor 8 is again inserted in flap L1 and released on its inner surface 75 and the suture thread S is passed outwards through the surface 71 to enable insertion of a fourth anchor 8 in flap L2 (on the inner surface 76) making a zigzag movement between the two flaps. Pulling the suture thread S through the anchors 8 enables the margins 72 and 73 of the wound to be approximated, thus closing it.

Various types of materials can be used to construct suture anchors, such as steel or other metals or alloys, plastic materials, composite materials, biologically inert materials or resorbable materials.

### Suture locking devices

The description will now be given of three practical forms of systems to lock the free end of suture thread as per the present invention. The locking devices may be used with different suturing systems, for example traditional suture systems or T-suture systems.

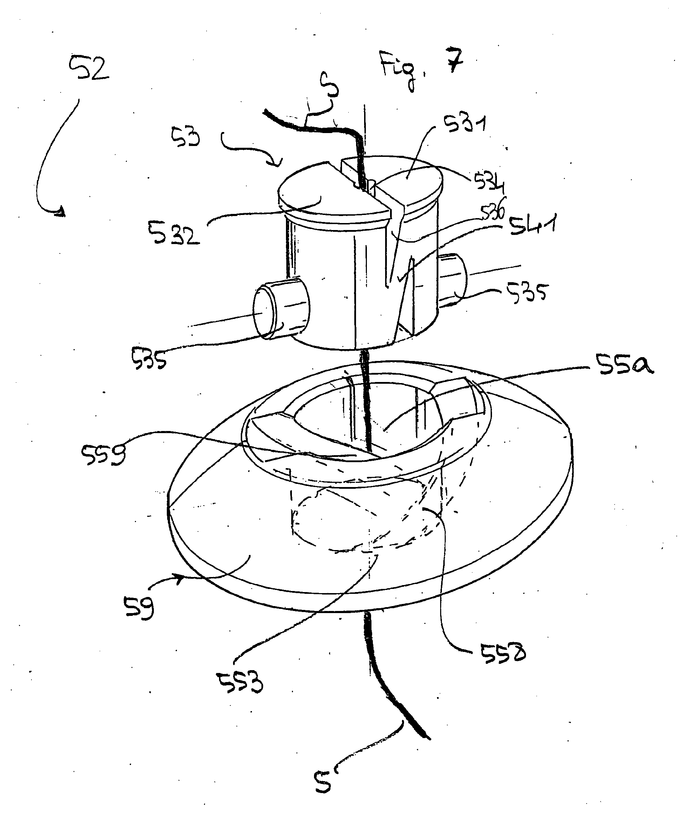

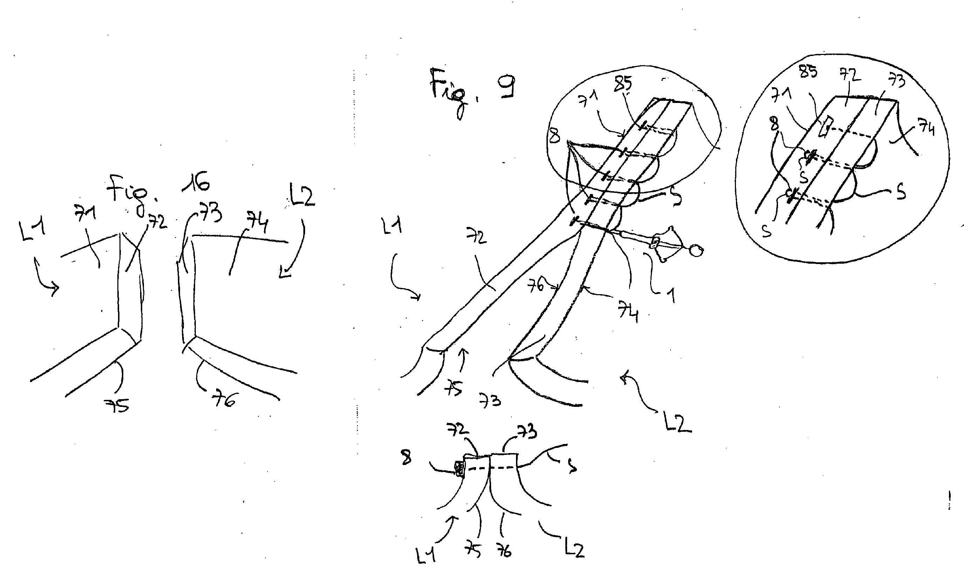

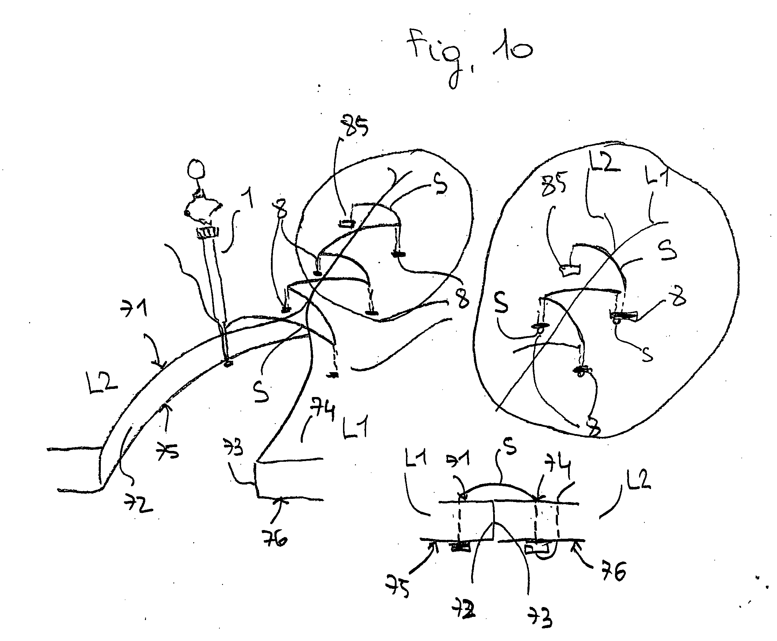

Figure 2 illustrates a first practical form of a locking device 50 known as the T form. The locking device 50 comprises a cylindrical anchor, approximately spindle shaped, in the centre of which is a through hole 55. The through hole 55 presents an initial truncated cone portion 551 and a second cylindrical portion 552. The cylindrical portion 552 of the hole has a diameter slightly inferior to that of the suture thread it is destined to house.

The suture thread S passes through said hole 55 such that it first passes through the truncated cone part 551 and subsequently through the cylindrical part 552. In this fashion the thread is partially blocked inside the hole 55 and its return movement is impeded. The surgeon then disposes the excess thread in one or more inverted rings 57 around the extremity of the cylindrical anchor, such that the proximal part of the thread is held in place by the distal part. The excess thread is then cut away.

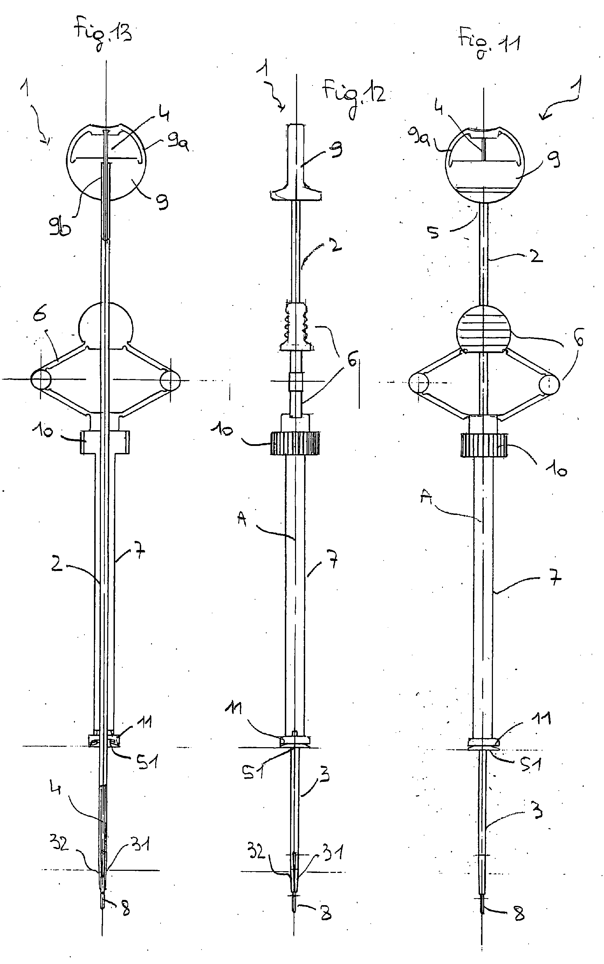

A second practical form of the locking device in accordance with the present invention, known as the button form, is illustrated in figures 3, 4 and 5, in which it is indicated overall with reference number 51.

The locking device 51 comprises a body having a cylindrical, ellipsoid or parallelepiped form. The body presents an upper portion 58 a lower portion 59 and a central through hole 55 the upper portion being displaced axially with regard to the lower portion. The axial through hole 55 has a diameter such as to enable its use with a suturing instrument such as for example the instrument 1 subject of the present invention and described below.

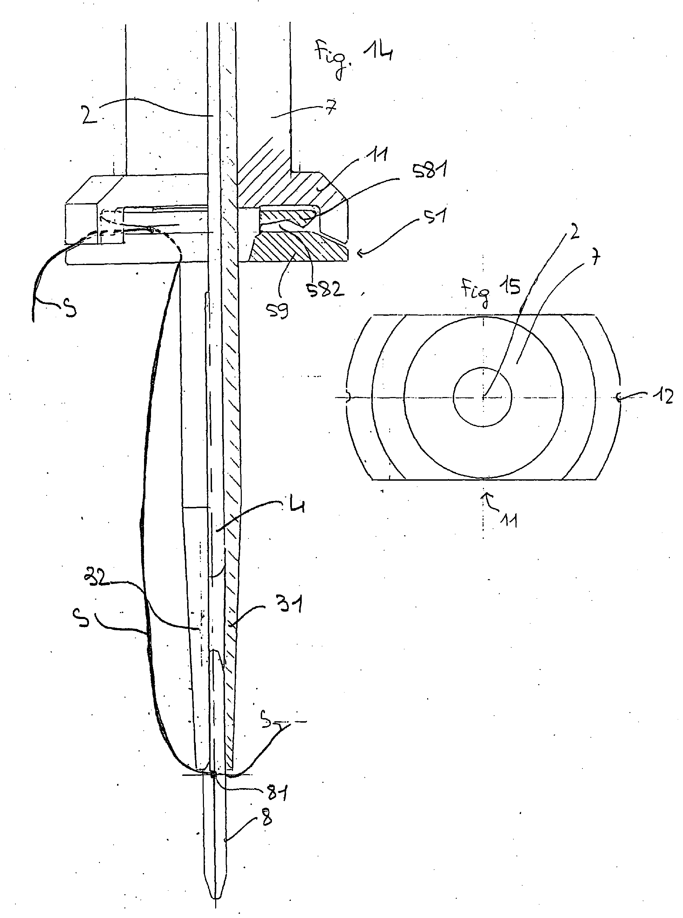

The upper portion 58 may be substantially disk-shaped or may comprise two or more radial wings 581 which delimit an anchorage section for the suture thread 582 between the upper portion 58 and the lower portion 59 of the device 51 (figure 4).

The device 51 also presents a groove or recess 555 in its lower portion 59 linked to and communicating with the hole 55 inside which the suture thread passes; the width of the groove 555 is slightly larger than that of the suture thread used. The suture thread passes through the groove 555 within the anchorage section 582 blocking it.

A variant of the locking device 51 illustrated in figure 6, provides for the body being composed of two components 589 and 598 bearing respectively the upper portion 58 and the lower portion 59 where such components are furnished with mutually co-operating portions 585 and 586 of a spring catch.

Once the above-described suturing procedure is completed, slight pressure with a suturing instrument such as the instrument 1 described below will cause the two portions 58 and 59 of the locking device 51 to engage, causing compression of the suture thread S such that its release is impeded.

A third practical form of the locking device as per the present invention, known as the screw form, is illustrated in figures 7 and 8, in which overall it is indicated with reference number 52.

The device 52 comprises a lower portion 59 substantially disk-shaped, cylindrical, truncated cone, ellipsoid or parallelepiped in shape, and is fitted with a central elliptical through hole 55a. On the inner walls of the hole 55 there are two channels 558 and 559 running along a 90° -120° helix path around the longitudinal axis of the surface of the hole 55 each ending at the distal face of the device 52 with a channel/indentation 553.

The device 52 comprises an upper portion, known as the screw 53. It comprises two cheeks 531 and 532 each provided with a groove 534 on the flat face passing through the centre 536 and having dimensions compatible with those of the suture thread S. These two cheeks 531 and 532 are connected together by two flexible sections 541. These sections 541 are of suitable size and connect the proximal part of one cheek to the corresponding distal part of the other.

Each cheek 531 and 532 bears on its external face and in the distal part, bayonet catches 535 which can slide in the channels 558 and 559 and engage in the indentation 553 situated on the inner surface of the lower portion 59 of the device 52.

The diameter of the groove 534 is slightly smaller than that of the suture thread used.

Once the suture anchor 8 is inserted the surgeon recovers the excess thread passing through the screw, 53.

Gentle pressure by the surgeon in the proximal-distal direction on the screw 53 causes a rotary movement to be impressed by the channels 558 and 559 inside the lower portion 59 of the locking device 52. This movement brings the two cheeks 531 and 532 together and in consequence compresses the suture thread in the groove 534 impeding its movement. The two cheeks 531 and 532 are locked in position when the bayonet catches 535 couple to the channels 553 of the inner distal surface of the device 52.

At this point the excess thread S is cut away.

To remove the locking device 52 it suffices to turn the screw 53 anticlockwise.

The said locking devices 50, 51 and 52 can take on various shapes and sizes without thereby falling outside the concept of the present invention. In particular, modifications of the external surfaces may be introduced to improve tissue adherence and furthermore shapes can be used that enable partial penetration inside the tissues.

For the construction of the locking devices 50 51 and 52 various different materials may be employed such as steel or other metals or alloys, plastic materials, composite materials, biologically inert materials and resorbable materials.

### Suturing instrument

The suturing devices 8 and the suture locking devices 51 and 52 described above can advantageously be used with an instrument for the suturing of tissues, subject of the present invention and illustrated in figure 11.

The proximal extremity of the T-suture instrument - indicated overall with reference 1 -- is destined to be manipulated by the surgeon, whereas the distal extremity is destined to exercise the functions proper to the instrument.

With reference to figures 11 to 15, the instrument 1 to apply T-sutures comprises a rigid tubular sheath 2 in which an operating rod 4 can slide axially.

The sheath 2 presents at the distal extremity two longitudinal cuts parallel to its principal axis A such that it has two opposed claws having a C-shaped section 31 and 32 (figure 14). The two claws 31 and 32 present at their distal extremity a slightly tapering configuration. Alternatively, the distal extremity of the sheath 2 may present an articulated hollow gripping system with two jaws (not illustrated).

A handgrip 9 is connected to the proximal extremity of the operating rod 4; said handgrip may take on different forms, for example pistol-type or syringe-type; its function is that of controlling the movement of the operating rod 4 inside the sheath 2.

In the case given as an example, the handgrip 9 is of a discoid shaped with the proximal or external part hollow, comprising a spring-born bow 9a which is connected in a central position to the proximal extremity of the rod 4. The rod 4 can otherwise slide axially, against the recall force exercised by the bow 9a in a hole 9b in the solid part of the handle 9.

The sheath 2 is in its turn mounted inside a second rigid sheath 7 whose proximal extremity is coupled with the sheath 2 through an elastic recall structure 6 in particular of the pantograph type. The second sheath 7 is free to rotate around the sheath 2 and its movement is controlled by means of a knurled wheel 10 integrally connected to the second sheath 7 in a position adjacent to the recall structure 6.

The distal extremity of the second sheath 7 presents at least one protuberance 11 extending radiantly from the second sheath 7 (figure 14). The protuberance 11 presents on its external perimeter a groove or recess 12 within which suture thread can run. The groove 12 has size slightly smaller than the diameter of the suture thread.

Penetration movement of the operating rod 4 inside the first sheath 2 causes the two claws 31 and 32 to open like the jaws of forceps. Retraction of the rod 4 brings the two claws 31 and 32 to close one upon the other with a pincer movement.

Alternatively, the distal extremity of the operating rod 4 may terminate with a T-shaped structure (not illustrated). In this case, the two arms of the T insert into the two cuts in the first sheath 2 between the two claws 31 and 32 causing them to open.

Whichever the specific action mechanism employed, the opening and closing movement of the two claws 31 and 32 enables a T-suture anchor, indicated with reference number 8, to be gripped and released.

A variant of the instrument 1 provides for one or more suturing anchors 8 to be housed inside the sheath 2.

The instrument 1 is also able to utilise suture thread locking devices, and in particular the device 51.

The distal portion of the instrument 1 is inserted inside the locking device 51 through the hole 55 (please refer to figures 4 and 14). The surgeon passes the proximal part of the suture thread through the groove 12 of the protuberance 11 of the second sheath 7 of the instrument 1. After the suture anchor 8 has been loaded, the surgeon recovers the part of suture thread S in excess. The distal part of the instrument, and in particular of the sheath 4 and the anchor 8 are again inserted through the tissue.

Operating the flexible bow of the handgrip 9 the rod 2 is made to operate, releasing the anchor 8. The excess suture thread is then partially recovered and the wheel 10 of the instrument 1 is rotated causing passage of the thread inside the anchorage section 582 of the locking device 51 causing the thread S to be blocked in place. At this point the instrument 1 is removed and excess thread is cut away.

The entire instrument 1 can take on different shapes and sizes depending on the use. For mini-invasive surgery the operating rod 4 the sheath 2 and the sheath 7 may be of considerable length in order to reach organs or tissues inside the human or animal body.

The operating rod 4 the sheath 2 and the sheath 7 can also be curved along their longitudinal axis.

### Fields of application

Applications in the surgical field of the devices described above are immediately evident to experts in the field; however, for descriptive and non-restrictive purposes the following may be listed: simple, continuous, open suturing, with T-sutures and locking devices or anchors in laparoscopy.

Among the types of sutures that can advantageously be applied using the T-suture device described in the present invention are various sutures, inguinal ring closure, suturing of prosthetic materials, repair of lacerations to the meniscus and others.

It is perfectly obvious to experts in the field that all the above-described devices may be produced and marketed as disposable instruments, multiple-use instruments or for single-patient use.

Naturally, the details of realisation and the practical forms can be widely varied with regard to what is described and illustrated here without coming outside the field of the present invention as defined in the attached claims.

## Claims

1. Suturing device, comprising a terminal T-shaped anchor (85) having a central portion to which is fixed one end of the suture thread (S) **characterised in that** it includes at least one intermediate anchor (8) having an elongated shape along a longitudinal axis (A), said intermediate anchor (8) being provided with at least one through hole (81) transversal with respect to said longitudinal axis (A) and through which said suture thread (S) extends and can run.

2. Suturing device according to claim 1, **characterised in that** it also includes a locking device (50, 51, 52) to which the free end of said suturing thread (S) is anchored.

3. Suturing device according to claim 1, **characterised in that** said at least one intermediate anchor (8) comprises a monolithic body (82) of spindle shape with rounded extremities and fitted with a single transversal through hole (81) having a diameter equal to or greater than the diameter of said suture thread (S).

4. Suturing device according to claim 2, **characterised in that** said locking device (50) comprises a body (502) elongated along the longitudinal axis (A') and fitted with a through hole (55) transversal to said longitudinal axis (A') having a first portion (552) with diameter smaller than the diameter of the suture thread (S).

5. Suturing device according to claim 4, **characterised in that** said transversal through hole (55) has a second portion (551) with truncated cone shape and with its greater diameter open to the external surface of the body (502) and the smaller diameter communicating with an inner extremity of said first portion (551).

6. Suturing device according to claim 2, **characterised in that** said locking device (51) comprises a lower portion (59), a central axial hole (55) and an upper portion (58) axially distant from said lower portion (59) such as to form an anchorage section (582) for the suture thread (S) between the lower portion (59) and the upper portion (58).

7. Suture device according to claim 6, **characterised in that** the upper portion (58) and the lower portion (59) are formed in a single monolithic body.

8. Suturing device according to claim 6, **characterised in that** said locking device (51) comprises a first and a second separate component (589, 598) bearing respectively the lower portion (59) and the upper portion (58), said separate components being provided with portions having mutually co-operating spring catches (585, 586).

9. Suturing device according to claim 6, **characterised in that** said upper portion (58) presents a conformation chosen from between:
-- a substantially disk-shaped structure, or
-- a structure with one or more radial wings (581).

10. Suturing device according to claim 6, **characterised in that** said lower portion (59) presents a groove (555) communicating with said hole (55).

11. Suturing device according to claim 2, **characterised in that** said locking device (52) comprises a first element (59) having an axial through hole (55a) and a second element (53) that can be fixed to the first element through bayonet catches (535), the second element (53) having two cheeks (531, 532) which approximate and grip the suture thread (S) locking the suture thread (S) between them by effect of fixing the second element (53) in the through hole (55 a) of the first element (59).

12. Suturing device according to claim 11, **characterised in that** said central through hole (55a) presents an elliptical shape.

13. Suturing device according to claim 12, **characterised in that** said hole (55a) presents on its walls two channels (558 and 559) running on the inner surface of the hole (55a) along its longitudinal axis with a 90° -120° helix pathway.

14. Suturing device according to claim 13, **characterised in that** said channels (558 and 559) terminate with respective indentations (553) on the distal face of said first element (59).

15. Suturing device according to claim 11, **characterised in that** said cheeks (531, 532) are connected together by two flexible sections (541).

16. Suturing device according to claim 15, **characterised in that** said flexible sections (541) connect the proximal extremity of one part (531 or 532) to the corresponding distal extremity of the other part (531 or 532).

17. Instrument to implant a suturing device according to one or more of the previous claims, comprising a first rigid tubular sheath (2) capable of housing at least one suture anchor (8) and an operating rod (4) sliding axially within said sheath (2), the movement of said rod (4) causing the expulsion of said anchor (8) into the tissue to be sutured, **characterised in that** said device (1) comprises a second rigid tubular sheath (7) external to the first sheath (2), said second sheath (7) being capable of housing a suture locking device (51) and capable of releasing said locking device (51).

18. Instrument according to claim 17, **characterised in that** the proximal extremity of said second sheath (17) is coupled to said first sheath (2).

19. Instrument according to claim 18, **characterised in that** said coupling is achieved through an elastic recall structure (6) in particular of the pantograph type.

20. Instrument according to any of the claims 17 to 19, **characterised in that** said second sheath (7) freely rotates around said first sheath (2) and said rotation is controlled by a wheel (10) integrally connected to said second sheath (7).

21. Instrument according to any of the claims 17 to 20, **characterised in that** the distal extremity of said second sheath (7) is provided with at least one protuberance (11) capable of coupling with said locking device (51).

22. Instrument according to claim 21, **characterised in that** said at least one protuberance (11) presents on its external perimeter a groove (12) capable of housing suture thread, S.

23. Instrument according to any of the claims 17 to 22, **characterised in that** said first sheath (2) presents at its distal extremity two opposed claws (31 and 32) with a "C" section.

24. Instrument according to any of the claims 17 to 23, **characterised in that** said first sheath (2) presents at its distal extremity a hollow articulated forceps gripping system.

25. Instrument according to any of the claims 17 to 24, **characterised in that** said rod (4) is connected to the handgrip (9) able to control the sliding movement of said rod (4) inside said first sheath (2) and to cause the opening and closing of said claws (31 and 32).

26. Instrument according to any of the claims 17 to 25, **characterised in that** said rod (4) terminates with a T-shaped structure, the arms of said T-shaped structure inserting between the two claws (31 and 32) of said first sheath (2).
